# EUROPEAN PATENT APPLICATION

(11) **EP 2 153 792 A1**
(43) Date of publication of application: **17.02.2010**
(21) Application number: 08740894.4
(22) Date of filing: 27.04.2008
(51) Int. Cl.: A61B 18/20, A61N 5/06

(54) **LASER DRILLING DEVICE, AND PROTECTIVE MEMBER AND CARTRIDGE FOR LASER DRILLING DEVICE**

(30) Priority: 27.04.2007 JP 2007119412
(71) Applicant: Arkray, Inc., Kyoto-shi, Kyoto 601-8045 (JP)
(72) Inventor: FUKUZAWA, Masahiro, Kyoto-shi Kyoto 601-8045 (JP); DOI, Shigeru, Kyoto-shi Kyoto 601-8045 (JP)
(74) Representative: HOFFMANN EITLE
(86) International application number: PCT/JP2008/058155
(87) International publication number: WO 2008/136450

(57) **Abstract**

The present invention is directed to a laser perforator apparatus 1 comprising a laser light oscillator 21 for outputting laser light to be applied to skin, and a protection member 4 disposed on an optical path before laser light output from the laser light oscillator reaches the skin. The protection member 4 covers a laser light outputting portion in the laser light oscillator 21 and includes a plurality of laser light transmission portions 42 permitting transmission of the laser light. Preferably, the protection member 4 is configured to be movable in a direction intersecting a transmission direction of the laser light.

## Description

### TECHNICAL FIELD

The present invention relates to a laser perforator apparatus for forming a perforation on the skin by laser light.

### BACKGROUND ART

Laser perforator apparatuses for forming a perforation on the skin by laser light are already practiced. The laser perforator apparatus enables sampling of a small amount of body fluid such as blood from a finger or the like by burning out the skin and blood vessel using laser light oscillated by a laser light oscillator. The body fluid thus sampled is utilized for own blood glucose determination using, for example, a simplified blood glucose meter. The perforator apparatus utilizing the laser light put to practical use has sufficient performance from a practical viewpoint of sampling of body fluid such as blood.

In sampling body fluid by the laser light, since smoke goes up from the skin burnt at laser light application, this smoke adheres in some cases to the laser light oscillator. When the smoke adheres to a lens of the laser light oscillator, output of the laser light being oscillated from the laser light oscillator is reduced and appropriate sampling of body fluid may become difficult.

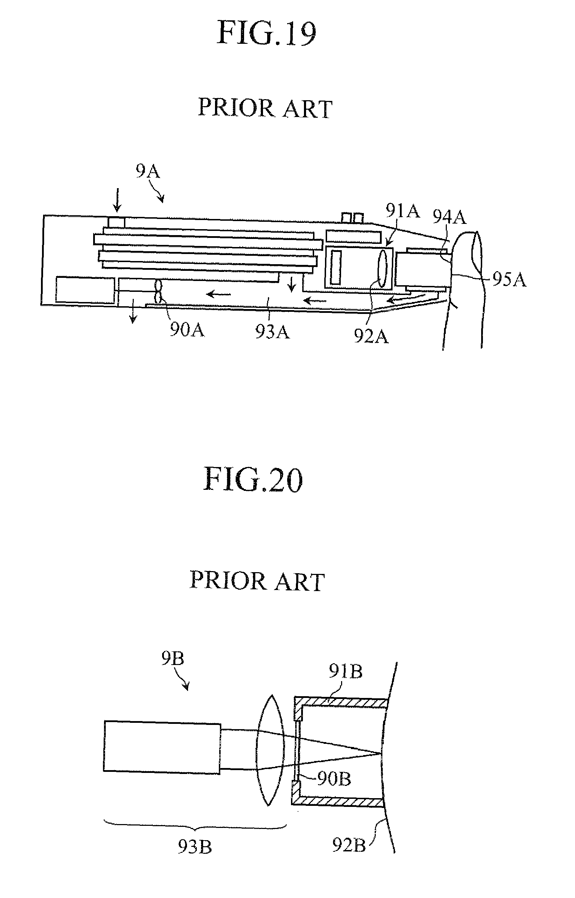

For eliminating such a drawback, perforator apparatuses 9A, 9B as shown in FIGS. 19 and 20 are made available.

The perforator apparatus 9A shown in FIG. 19 includes a fan device 90A. With this perforator apparatus 9A, an airstream is generated around perforating site by utilizing air flow caused by the fan device 90A, and it is possible to prevent the smoke from reaching a lens 92A of a laser light oscillator 91A.

With a perforator apparatus 9B shown in FIG. 20, a cover 91B having a member 90B that can allow transmission of laser light is mounted. In this perforator apparatus 9B, skin 92B and laser light oscillator 93B are isolated by a cover 91.

However, with the perforator apparatus 9A shown in FIG. 19, the perforator apparatus 9A becomes large in size and disadvantageous from cost viewpoint, because the fan device 90A and an air flow path 93A are required. Further, with the perforator apparatus 9A, there is a danger of infection if it makes contact with the skin 94A and site 95A is contaminated with the blood.

Meanwhile, with the perforator apparatus 9B shown in FIG. 20, replacement of the cover 91B is necessary for every perforating manipulation, thereby increasing burdens of a user.

Patent Document 1: US5947957A
Patent Document 2: WO98/47435

### DISCLOSURE OF THE INVENTION

### PROBLEMS TO BE SOLVED BY THE INVENTION

An object of the present invention is to suppress adhesion of smoke or the like to the laser light oscillator while reducing operational burdens of the user.

Another object of the present invention is to suppress increase in size of the perforator apparatus and increase in costs.

Still another object of the present invention is to suppress adhesion of body fluid such as blood to a site contacting the skin in the perforator apparatus and to reduce a danger of infection.

### MEANS FOR SOLVING THE PROBLEM

In a first aspect of the present invention, such a laser perforator apparatus is provided which includes a laser light oscillator for outputting laser light to be applied to skin and a protection member disposed on an optical path before laser light being output from the laser light oscillator reaches the skin, wherein the protection member covers laser light outputting region in the laser light oscillator and includes a plurality of laser light transmission portions permitting transmission of the laser light.

Preferably, the protection member is configured to be movable in a direction intersecting a transmission direction of the laser light.

The protection member is designed, for example, to be movable in a linear fashion. If this is the case, the plurality of laser light transmission portions are preferably disposed so as to form a row shape in the direction of movement of the protection member.

The protection member is arranged, for example, in the movement direction and has a plurality of individual regions having the laser light transmission portions. The individual regions may be configured to be separable at a fragile part provided between adjacent individual regions.

The laser perforator apparatus of the present invention preferably further includes a carrier device for movement of the protection member. The carrier device includes, for example, a rotating body having an engaging member for engagement to an engaged member being formed to the protection member. The individual region is separated at the fragile part by, for example, turning the rotating body with the engaging member being engaged with the engaged member.

The plurality of laser light transmission portions may be disposed side by side on the same circumference. In this case, the protection member is formed in, for example, rotatable disk shape. The plurality of laser light transmission portions in this case is preferably disposed side by side in a circumferential direction of the protection member.

The laser perforator apparatus of the present invention further includes, for example, a cap for retaining the protection member movable. The cap preferably includes an aperture for exposing the laser light transmission portion.

The protection member is provided on the periphery of the laser light transmission portion and may further include a convex portion for exposure via the aperture. In this case, the aperture is preferably formed to expose the convex portion wholly. The convex portion is formed, for example, in a ring pattern enclosing the laser light transmission portion.

The protection member may be formed in a film shape to allow transmission of the laser light. The protection member in this case is contained in a state, for example, being rolled-up in a case.

The laser perforator apparatus of the present invention may further include a supporting member for supporting the case. In this case, the protection member is configured such that at least a part thereof is slid in a direction intersecting the movement direction of the supporting member by moving, for example, the supporting member.

In a second aspect of the present invention, such a laser perforator apparatus is provided which includes a laser light oscillator for outputting laser light to be applied to skin and a plurality of protection members for protection of the laser light oscillator, wherein the protection member is configured to be movable from a position that does not cover a laser light outputting region in the laser light oscillator to a position that covers the outputting region.

The plurality of protection members are contained, for example, in a state of being formed in a plate-like shape and laminated in a thickness direction.

The laser perforator apparatus of the present invention further includes a case for containing, for example, the plurality of protection members. The case is preferably made detachable in the laser perforator apparatus.

The laser perforator apparatus of the present invention further includes a housing in which, for example, the laser light oscillator is contained and a cap for covering the housing. The case in this case is made detachable with respect to the cap.

In a third aspect of the present invention, in the laser perforator apparatus including the laser light oscillator for outputting laser light to be applied to skin, protection members, which are disposed on the optical path before laser light being output from the laser light oscillator reaches the skin, cover the laser light outputting region in the laser light oscillator, and include a plurality of laser light transmission portions that allow transmission of the laser light, are provided.

The plurality of laser light transmission portions is disposed, for example, side by side on the same straight line.

The protection member of the present invention includes a plurality of individual regions having, for example, the laser light transmission portions. Each of the individual regions is preferably made separable at the fragile part provided between adjacent individual regions.

The plurality of laser light transmission portions may be disposed side by side on the same circumference.

In a fourth aspect of the present invention, a cartridge which is used in the laser perforator apparatus having the laser light oscillator for outputting laser light to be applied to the skin and which includes the protection members disposed on the optical path before laser light being output from the laser light oscillator reaches the skin is provided, wherein the protection members are formed in a film shape to allow transmission of the laser light.

The protection members are contained in a state, for example, of being rolled-up in the case.

In a fifth aspect of the present invention, a cartridge which is used in the laser perforator apparatus having the laser light oscillator for outputting laser light to be applied to the skin and which contains a plurality of protection members for protection of the laser light oscillator is provided.

The plurality of protection members are contained in the case, for example, in a state of being formed in a plate-like shape and laminated in the thickness direction.

### BRIEF DESCRIPTION OF THE DRAWINGS

FIG. 1 is a perspective view of the laser perforator apparatus relating to a first embodiment of the present invention.
FIG. 2 is a perspective view showing a state where the cap is removed in the perforator apparatus shown in FIG. 1.
FIG. 3 is a sectional view taken along line III-III of FIG. 1.
FIG. 4 is a sectional view taken along line IV-IV of FIG. 2.
FIG. 5 is a sectional view in which main portions of FIG. 4 are enlarged.
FIG. 6 is a plan view showing protection members used in the laser perforator apparatus shown in FIG. 1.
FIG. 7 is a sectional view taken along line VII-VII of FIG. 6.
FIG. 8 is a sectional view in which main portions of the protection member shown in FIG. 6 are enlarged.
FIG. 9A through FIG. 9D are pattern diagrams in which main portions of the laser perforator apparatus are shown in a simplified form for description of operations of the laser perforator apparatus shown in FIG. 1.
FIG. 10A through FIG. 10D are pattern diagrams in which main portions of the laser perforator apparatus are shown in a simplified form for description of operations of the laser perforator apparatus shown in FIG. 1.
FIG. 11 is a sectional view showing main portions of the laser perforator apparatus relating to a second embodiment of the present invention.
FIG. 12 is a plan view of the protection members used in the laser perforator apparatus shown in FIG. 11.
FIG. 13 is a sectional view taken along line XIII-XIII of FIG. 12.
FIG. 14 is a sectional view showing main portions of the laser perforator apparatus relating to a third embodiment of the present invention.
FIG. 15 is a perspective view of the cartridge used in the laser perforator apparatus shown in FIG. 14.
FIG. 16A through FIG. 16C are sectional views for description of operations of the laser perforator apparatus shown in FIG. 14.
FIG. 17A through FIG. 17C are sectional views for description of operations of the laser perforator apparatus shown in FIG. 14.
FIG. 18 is a sectional view showing main portions of the laser perforator apparatus relating to a fourth embodiment of the present invention.
FIG. 19 is a sectional view for description of one example of the conventional laser perforator apparatus.
FIG. 20 is a pattern diagram showing the conventional laser perforator apparatus in a simplified form for description of another example.

### DESCRIPTION OF REFERENCE SIGNS

- 1, 5, 6, 8: Laser perforator apparatus
- 21: Laser light oscillator
- 22: Carrier deice
- 27: Rotating body
- 28: Hook (engaging member) of (rotating body)
- 3: Cap
- 32: Aperture
- 4, 50: Protection members
- 40: Split groove (fragile part) of (protection member)
- 41: Individual region (of protection member)
- 42, 52: Laser light transmission portion of (protection member)
- 43, 52A: Ring pattern wall (convex portion) of (protection member)
- 44: Slit (engaged member) of (protection member)
- 60: Cartridge
- 61: Case of (cartridge)
- 62: Sheet (protection member)
- 80: Cartridge
- 81: Case of (cartridge)
- 82: Protection member

### BEST MODE FOR CARRYING OUT THE INVENTION

Referring now to the drawings, preferred embodiments of the present invention will be described in detail below.

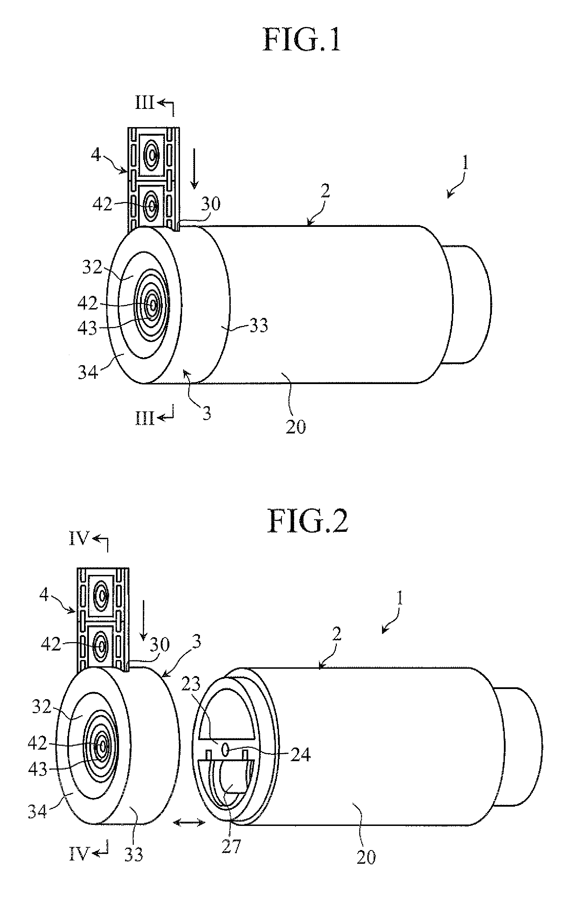

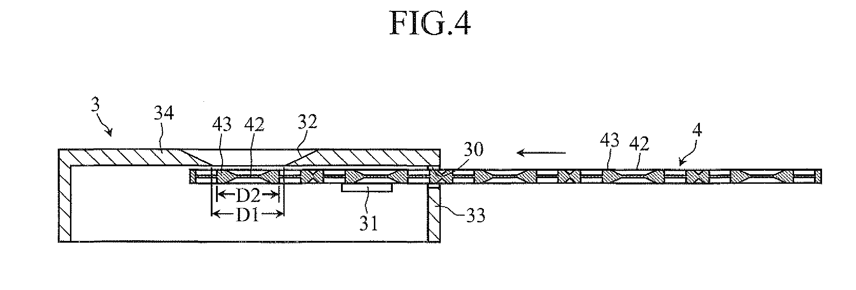

First, a first embodiment of the present invention will be described referring to FIG. 1 through FIG. 10.

The perforator apparatus 1 shown in FIGS. 1 and 2 is for sampling of body fluid such as blood from the skin by applying laser light to the skin. This perforator apparatus 1 includes a device body 2, a cap 3 and a protection member 4.

As shown in FIG. 1 through FIG. 3, the device body 2 includes a housing 20, a laser light oscillator 21 and a carrier device 22.

The housing 20 contains the laser light oscillator 21 and the carrier device 22 and includes a guide 23. The guide 23 defines a movement path of the protection member 4 and positions a laser light transmission portion 42 in the protection member 4, which will be described later, at a focusing position of the laser light from the laser light oscillator 21. This guide 23 has a penetrating hole 24 to permit transmission of the laser light.

The laser light oscillator 21 oscillates laser light to be applied to the skin and includes a laser light oscillation source 25 and a lens 26. The laser light oscillation source 25 comprises YAG laser or the like. The lens 26 focuses laser light from the laser light oscillation source 25 onto the skin surface.

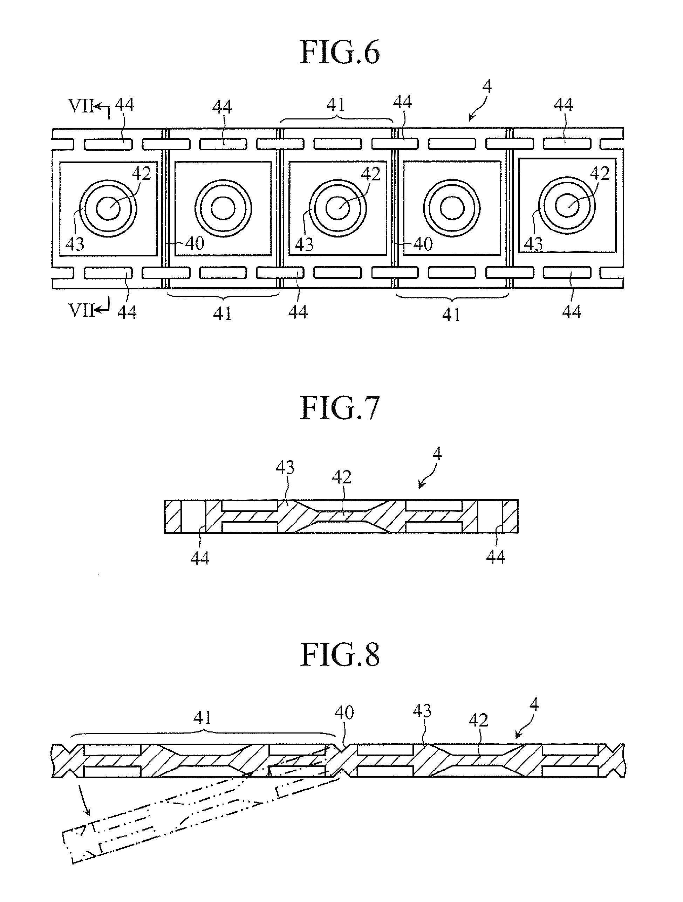

The carrier device 22 moves the protection member 4 by a predetermined distance in the direction of the laser light transmission portions 42 arranged side-by-side which will be described later. This carrier device 22 includes a rotating body 27. The rotating body 27 is rotatable in the arrow direction in the figure (counterclockwise) by turning force from a motor (not shown) or the like and includes four hooks 28. When the protection member 4 is moved by a target distance (distance corresponding to the length dimension of an individual region 41 which will be described later), turning operation of the rotating body 27 is controlled by control means (not shown) to turn every 90 degrees. Each of hooks 28 is engaged with a slit 44 in the protection member 4 which will be described later and is used for cutting of the individual region 41 in the protection member 4 (see FIG. 6 through FIG. 8) which will be described later. The four hooks 28 are disposed every 90 degrees apart in the circumferential direction of the rotating body 27 so as to be protruded from the circumferential surface of the rotating body 27.

As shown in FIG. 1 through FIG. 5, the cap 3 is mounted to the device body 2 (housing 20) and retains the protection member 4. This cap 3 is formed in a cylindrical shape and includes an insertion port 30, a guide 31 and an aperture 32.

The insertion port 30 is used when the cap 3 is caused to retain the protection member 4 and permits movement of the protection member 4. This insertion port 30 is formed as a penetrating hole in a rectangular shape in a circumferential wall part 33 of the cap 3.

The guide 31 plays a role of retaining the protection member 4 and defines a movement path of the protection member 4 together with the insertion port 30. This guide 31 is formed as a flange protruding inwardly from the circumferential wall part 33 of the cap 3.

The aperture 32 is such a portion to which a fingertip or the like is placed when perforation is formed on the skin. This aperture 32 exposes laser light transmission portion 42 of the protection member 4 and a ring pattern wall 43 which will be described later and is formed as a penetrating hole in a taper shape in a circular wall 34 of the cap 3. Minimum diameter D1 of the aperture 32 is greater than the outside diameter D2 of the ring pattern wall 43 and is set to, for example, 10 to 20 mm.

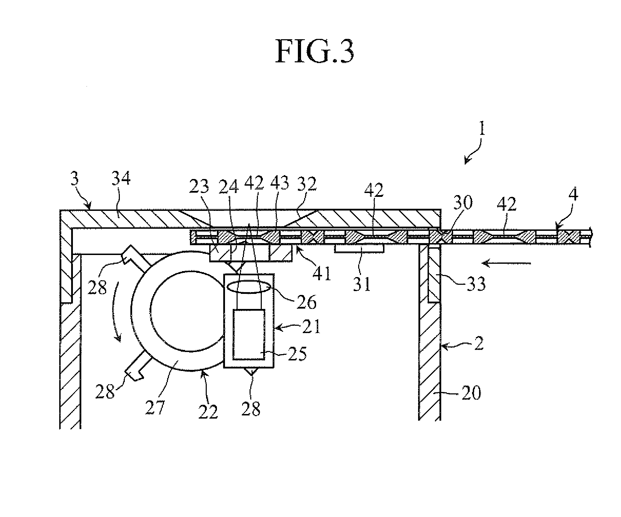

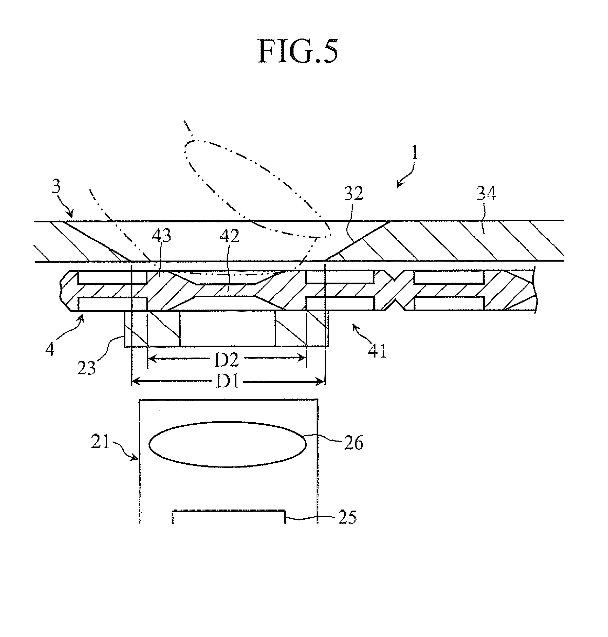

As shown in FIG. 5 through FIG. 8, the protection member 4 suppresses primarily adhesion of smoke generated when laser light is applied to the skin to the laser light oscillator 21 and is configured as a disposable material. This protection member 4 is formed in a rectangular shape as a whole and has a plurality of individual regions 41 (five in the figure) arranged in the movement direction of the protection member 4. The individual region 41 located at the most downstream in the direction of movement of the protection member 4 is disposed so as to cover the light outputting region of the laser light oscillator 21 (see FIGS. 3 and 5). Each of the individual regions 41 is normally moved by a distance corresponding to the length dimension of the individual regions 41 for every one-time laser light application. A split groove 40 is formed between adjacent individual regions 41 and each of the individual regions 41 is capable of being cut at the split groove 40.

Each of the individual regions 41 has a laser light transmission portion 42 and the ring pattern wall 43. That is, the plurality of laser light transmission portions 42 and the ring pattern wall 43 are provided so as to form a row shape in the direction of movement of the protection member 4. The laser light transmission portions 42 permit transmission of the laser light oscillated by the laser light oscillator 21 and are defined in a circular shape. The thickness of this laser light transmission portions 42 is set to, for example, from 0.1 to 0.5 mm. Meanwhile, the diameter of the laser light transmission portions 42 is set to, for example, from 2 to 5 mm. As a matter of course, the shape of the laser light transmission portions 42 is not limited to the circular shape and may take other shapes such as a rectangle.

The ring pattern wall 43 is a portion that is exposed by the aperture 32 of the cap 3 (see FIGS. 1 and 5) and is formed so as to enclose the laser light transmission portions 42. Since the ring pattern wall 43 is exposed from the aperture 32, when a fingertip or the like is placed on the aperture 32, the skin makes contact with the ring pattern wall 43.

The protection member 4 further includes a plurality of slits 44. The plurality of slits 44 are formed into a row shape at both side edges of the protection member 4. These slits 44 are portions where the hook 28 of the rotating body 27 in the carrier device 22 is engaged (see FIG. 3). When the rotating body 27 is turned in a state where the hook 28 is engaged with the slit 44, it is possible to move the protection member 4 and the individual region 41 can be cut. Such a protection member 4 can be formed by resin molding using, for example, a transparent resin material. As the transparent resin material, for example, polymethylmethacrylate (PMMA), polystyrene (PS), polycarbonate (PC) or polyethylene terephthalate (PET) may be used.

Next, operations of the laser perforator apparatus 1 will be described.

As shown in FIGS. 1, 3, 5, and 9A, when the cap 3, which is caused to retain the protection member 4, is mounted to the device body 2, the individual region 41 of the protection member 4 is situated opposite the guide 23 of the housing 20. In this case, the hook 28 of the rotating body 27 is engaged with the slits 44 positioned at both sides of the individual region 41 opposing the guide 23.

When a perforation is formed on the skin with the laser perforator apparatus 1, laser light may be oscillated from the laser light oscillation source 25 while, for example, a fingertip is placed on the aperture 32 of the cap 3. The laser light oscillated from the laser light oscillation source 25 is focused with the lens 26 and is then applied to the skin after transmitted through the laser light transmission portion 42 of the protection member 4. When the laser light is applied to the skin, skin and blood vessel are burnt by energy of the laser light resulting in a perforation, and bleeding of body fluids such as blood from the skin takes place.

In the perforator apparatus 1, the light outputting portion of the laser light oscillator 21 is covered by the individual region 41 of the protection member 4. Therefore, smoke generated when laser light is applied to the skin hardly reaches the light outputting portion of the laser light oscillator 21.

Further, in the perforator apparatus 1, the laser light transmission portion 42 of the protection member 4 and the ring pattern wall 43 are exposed by the aperture 32 of the cap 3. Therefore, at laser light application, skin of such as a fingertip is in contact with the ring pattern wall 43 surrounding the laser light transmission portion 42. In the meantime, laser light is applied to the skin after transmitted through the laser light transmission portion 42 being enclosed by the ring pattern wall 43. In other words, a perforation forming site (fluid bleeding portion) on the skin is a region enclosed by the ring pattern wall 43. For this reason, when bleeding of body fluid from the skin occurs due to laser light application, a possibility of adhesion of the body fluid to the interior of the aperture 32 of the cap 3 is reduced.

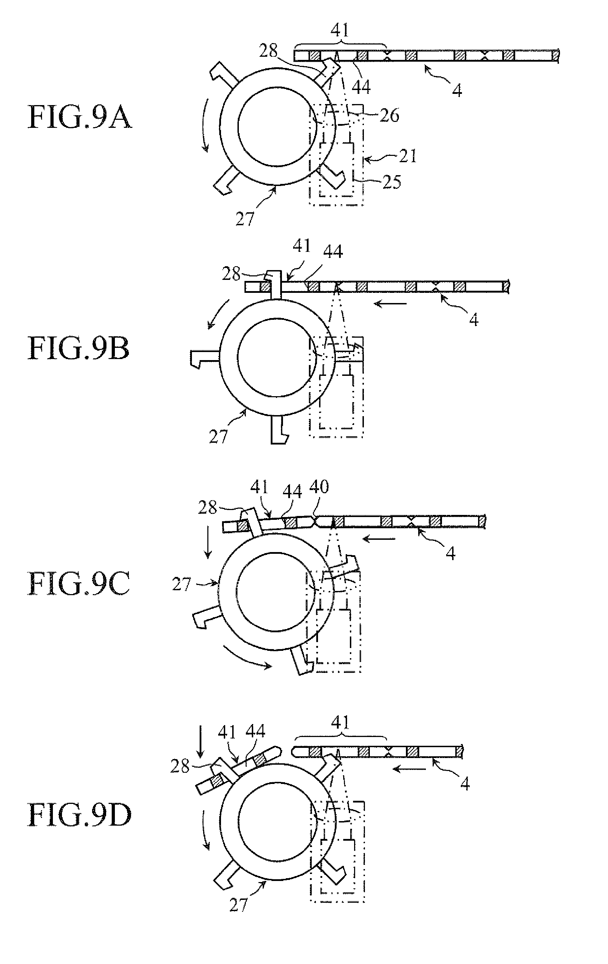

Upon completion of formation of a perforation to the skin, with the perforator apparatus 1, the rotating body 27 is turned by 90 degrees as illustrated in FIG. 9A through FIG. 9D to move the protection member 4 by a distance corresponding to the length dimension of the individual region 41. With these manipulations, the used individual region 41 is separated from the protection member 4 and an individual region 41 that is different from the previous one is moved to a position corresponding to the guide 23 (see FIGS. 2 and 3) of the device body 2 (housing 20).

More specifically, as shown in FIGS. 9A and 9B, since the hook 28 of the rotating body 27 is engaged with the slits 44 positioned at both sides of the individual region 41 in the protection member 4, when the position of the hook 28 is changed on a circumferential trajectory by rotation of the rotating body 27, the protection member 4 is moved. Since the movement path is defined by the guide 31 of the cap 3 and by the guide 23 of the housing 20 (see FIG. 3), the protection member 4 tends to go straight ahead in the direction of the arrow in the figure (left direction).

In contrast, since the hook 28 moves on the circumferential trajectory, the hook 28 tends to move the protection member 4 downwardly in a state where the hook 28 is caught by the slit 44 as shown in FIG. 9C. As a result, stress concentration in the protection member 4 occurs at the split groove 40 and the individual region 41 with the engaged hook 28 is separated at the split groove 40 as shown in FIG. 9D. The separated individual region remains being engaged with the hook 28.

In the meantime, movement of the protection member 4 results in movement of the individual region 41 that is different from the previous one to a position corresponding to the guide 23 of the device body 2 (housing 20). With these manipulations, the light outputting portion of the laser light oscillator 21 will be covered by a new individual region 41. As a result, even if the individual region 41 used previously is contaminated by body fluid or the like, a hygienic individual region 41 free from adhesion of blood or the like can be used when a perforation is formed next time by the laser light oscillator 21. Therefore, if the protection member 4 including the plurality of individual regions 41 is used to move the protection member 4 (individual region 41) every time a perforation is formed, possibility of infection or the like can be avoided as much as possible.

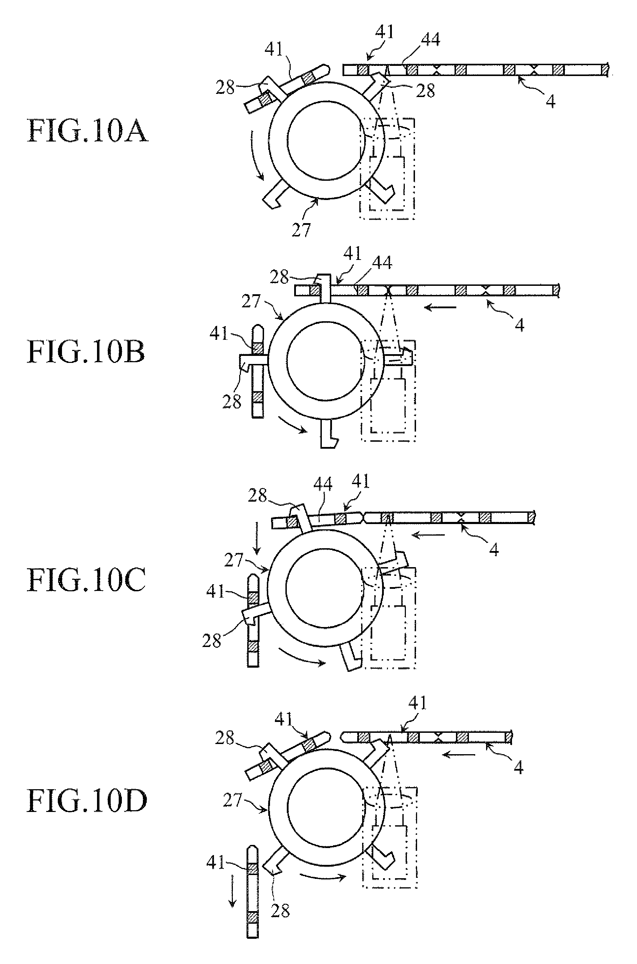

As shown in FIG. 10A through FIG. 10D, when the rotating body 27 is turned again by 90 degrees after formation of a next perforation, the protection member 4 is moved by a distance corresponding to the length dimension of the individual region 41 and the used individual region 41 is separated from the protection member 4. Meanwhile, the individual region 41 remained being engaged with the hook 28 is disengaged from the hook 28 and separated from the rotating body 27. The separated individual region 41 is contained in a disposal box provided in the housing 20 or disposed of from a disposal port provided in the housing 20.

If the used individual region 41 is separated as mentioned above, there is no need for the user to separate the used individual region 41 and accordingly, not only burdens of the user are reduced, but also the need of the user to touch the individual region 41 is reduced, and a hygienically excellent feature is thus obtained.

Here, if all the individual regions 41 in the protection member 4 are used and a new protection member 4 is caused to retain the cap 3, it is possible to maintain such a state where the light outputting portion of the laser light oscillator 21 is covered by the individual region 41 of the protection member 4. That is, when the protection member 4 is used, the protection member 4 need not be replaced every time the manipulation of perforation formation is conducted, and a new protection member 4 is simply mounted after the manipulation of formation of a predetermined number of perforations is conducted, and burdens of the user to replace the protection member 4 are reduced.

In addition, with the method using the protection member 4, there is no need to provide a fan device to the perforator apparatus 1 and to form a flow path for movement of the air, and the perforator apparatus 1 need not be made large resulting in cost-effective features.

Start of movement of the protection member 4 (turning of rotating body 27) may be set such that the user presses a lever or a button provided to the perforator apparatus or automatic operation takes place in the perforator apparatus 1. It is also possible to set it such that the protection member 4 is moved after formation of a plurality of perforations instead of moving the protection member 4 for every one-time formation of the perforation.

The protection member 4 need not necessarily be configured such that the individual region 41 is separable at the split groove 40, but may be configured such that the protection member is disposed of without splitting upon completion of use of all the individual regions in the protection member, and the carrier device 22 for movement of the protection member 4 is not limited to the one described previously.

Further, the ring pattern wall surrounding the laser light transmission portion may be one partly cut or may be omitted. When the ring pattern wall is omitted, the protection member may be formed in a film shape.

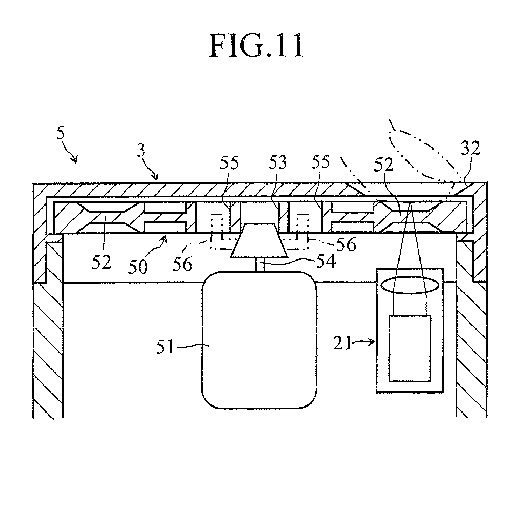

Next, referring to FIG. 11 through FIG. 13, a second embodiment of the present invention will be described. Like parts are denoted in these figures by the same reference numerals as used in the first embodiment described previously, and the description thereof is not given redundantly.

A laser perforator apparatus 5 shown in FIG. 11 uses a protection member 50 in a disk shape and includes a motor 51.

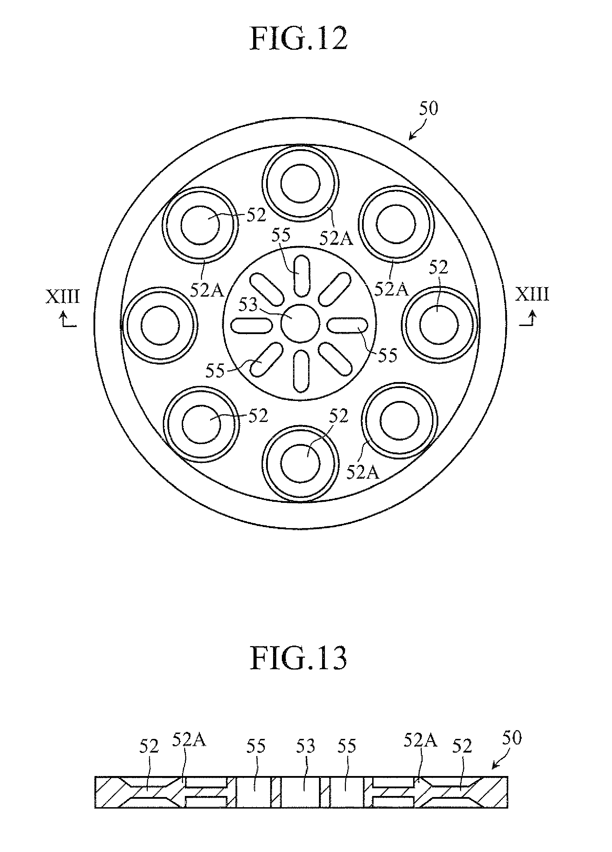

The protection member 50 is rotatably retained in the cap 3 and a plurality of laser light transmission portions 52 as shown in FIGS. 12 and 13 (eight in the figure) are disposed side-by-side in a circumferential direction. These laser light transmission portions 52 are disposed on the same circumference with predetermined intervals and are surrounded by a ring pattern wall 52A.

The protection member 50 of this sort can be formed by resin molding using, for example, a transparent resin material. As the transparent resin material, for example, polymethylmethacrylate (PMMA), polystyrene (PS), polycarbonate (PC) or polyethylene terephthalate (PET) may be used.

In the meantime, as shown in FIG. 11, the aperture 32 of the cap 3 is provided on a circumference where the laser light transmission portions 52 are disposed. That is, each of the plurality of laser light transmission portions 52 and ring pattern walls 52A can be exposed from the aperture 32 of the cap 3 and it is possible to change sequentially the laser light transmission portions 52 to be exposed from the aperture 32 by turning the protection member 50.

The motor 51 exerts a turning force to the protection member 50 utilizing a penetrating hole 53 of the protection member 50. Turning of a shaft 54 of this motor 51 is controlled by control means (not shown). Since eight laser light transmission portions 52 are provided in the protection member 50, the rotational angle of the shaft 54 is controlled every 45 degrees. In other words, positions of the plurality of laser light transmission portions 52 are changed by rotational output from the motor 51 and are shifted sequentially to the position corresponding to the aperture 32 of the cap 33.

Here, turning of the protection member 50 may be performed automatically in the perforator apparatus 5 being interlocked with perforating operation or performed by the user who manipulates a lever or button provided to the perforator apparatus 5. Further, the protection member 50 may be turned every one-time formation of the perforation or the protection member 50 may be turned every formation of the plurality of perforations.

With the perforator apparatus 5 as mentioned, by turning the protection member 50, the light outputting portions of the laser light oscillator 5 will be covered by different laser light transmission portion 52. For this reason, even if the laser light transmission portion 52 used before is contaminated by adhesion of body fluid, it is possible to use a hygienic laser light transmission portion 52 when a perforation is formed next time by the laser light oscillator 21, and accordingly, possibility of infection or the like can be avoided as much as possible.

Further, the protection member 50 may be replaced with a new one after all the laser light transmission portions 52 are utilized, thereby reducing replacement frequency of the protection member 50 and reducing burdens of the user for replacement of the protection member 50.

In the meantime, although the perforator apparatus 5 is configured to input turning force of the motor 51 to the protection member 50 using the penetrating hole 53, turning force of the motor 51 may be input utilizing an elongate hole 55 provided around the penetrating hole 53. In this case, as shown in FIG. 11, a click 56 (phantom two-dot chain line in FIG. 11) coupled with the shaft 54 of the motor 51 may simply be engaged with the elongate hole 55.

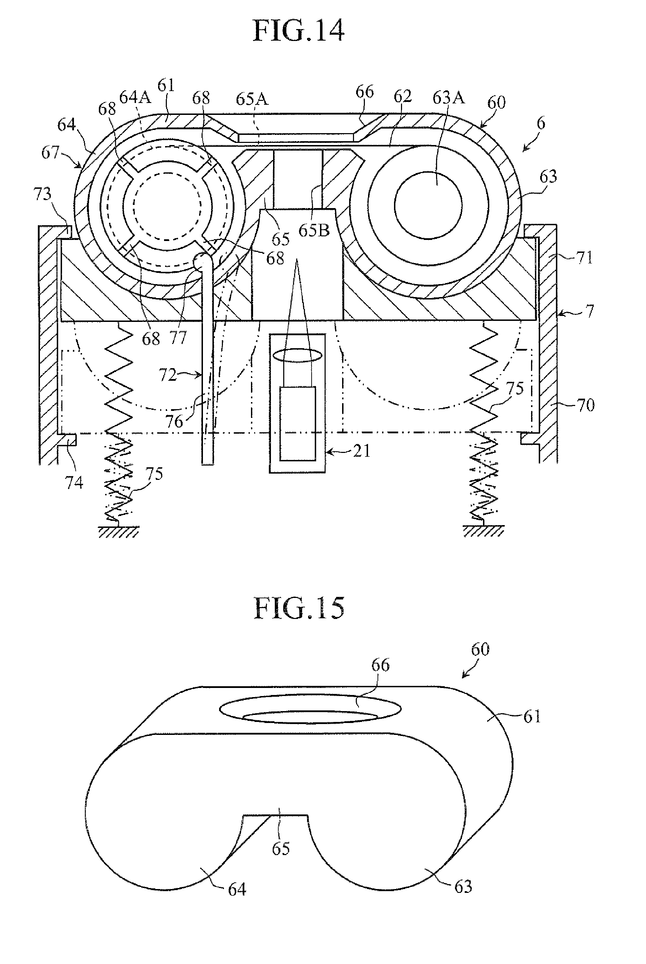

Next, a third embodiment of the present invention will be described referring to FIG. 14 through FIG. 17.

A perforator apparatus 6 shown in FIG. 14 protects the laser light oscillator 21 from smoke or the like using a cartridge 60 and further includes a device body 7.

As shown in FIGS. 14 and 15, the cartridge 60 contains a film 62 rolled-up in a roll shape inside a case 61. The film 62 is formed by materials which allow transmission of the laser light, for example, polyethylene terephthalate (PET), vinyl chloride, polypropylene (PP) or polyethylene (PE) to a thickness from 10 to 250 µm.

The case 61 includes roll containing portions 63, 64, a joining portion 65 and an aperture 66, and it is possible to move the film 62 by passing through the joining portion 65 from the roll containing portion 63 toward the roll containing portion 64.

The roll containing portion 63 contains rotatably a roll 63A on which the unused part of the film 62 is wound.

The roll containing portion 64 contains rotatably a roll 64A on which the used part of the film 62 is wound and is rotatable in one direction only (counterclockwise in the figure) by a one-way clutch or the like. An inputted member 67 is mounted to the roll 64A. The inputted member 67 is formed in a circular shape as a whole and is rotatable together with the roll 64A in an integrated fashion. This inputted member 67 has a plurality of ribs 68 (four in the figure) extending in a radial direction of the inputted member 67. The plurality of ribs 68 are provided in a circumferential direction of the inputted member 67 spaced apart with predetermined intervals (corresponding to 90 degrees in the circumference direction). These ribs 68 interfere with an inputting member 72 which will be described later and the inputted member 67 is set to be turned by a load input by the inputting member 72.

The diameter of the inputted member 67 is set according to the movement distance of the film 62 corresponding to the rotating pitch of the inputted member 67. For example, when the inputted member 67 is turned every 90 degrees and the movement distance of the film 62 is set equal to or larger than the maximum diameter of the aperture 66 of the case 61, the diameter of the inputted member 67 is set equal to or 1.25 times larger than the maximum diameter of the aperture 66. More specifically, when the maximum diameter of the aperture 66 of the case 61 is set to around 10 to 20 mm, the diameter of the inputted member 67 is set to around from 10 to 25 mm.

The joining portion 65 connects the roll containing portion 63 and the roll containing portion 64 and has a space 65A and a penetrating hole 65B. The space 65A permits movement of the film 62 and is communicated with the internal space of the roll containing portions 63, 64. The penetrating hole 65B permits transmission of laser light oscillated from the laser light oscillator 21, communicated with the space 65A and is positioned immediately above the laser light oscillator 21.

The aperture 66 is a portion onto which a fingertip or the like is placed when a perforation is formed on the skin. This aperture 66 is formed as a penetrating hole in a taper shape and exposes a portion positioned at the joining portion 65 in the film 62. The exposed portion in the film 62 is positioned immediately above the laser light oscillator 21 and covers the light outputting portion in the laser light oscillator 21. In the meantime, since the film 62 is formed by materials that permit transmission of laser light, the exposed portion constitutes a laser light transmission portion.

As shown in FIG. 14, the device body 7 includes a housing 70, a supporting block 71, the inputting member 72 and the laser light oscillator 21.

The housing 70 contains the supporting block 71, the inputting member 72 and the laser light oscillator 21 and includes stoppers 73, 74. The stoppers 73, 74 define movement range of the supporting block 71.

The supporting block 71 is a portion onto which the cartridge 60 is placed and is movable reciprocally in up and down directions. This supporting block 71 is biased upwardly by a coil spring 75. That is, the supporting block 71 is engaged with the stopper 73 while no load is input. In contrast, when a load is input downwardly, the coil spring 75 is compressed and the supporting block 71 moves downwardly. A maximum downward movement distance of the supporting block 71 is defined by the stopper 74. When load input to the supporting block 71 is released, the coil spring 75 returns by elasticity and the supporting block 71 returns to a position that interferes with the stopper 73.

The inputting member 72 inputs a load to the inputted member 67 and turns the inputted member 67 and eventually turns the roll 64A. This inputting member 72 has an elastic member 76 and an engaging member 77. The elastic member 76 allows positional displacement of the engaging member 77. The engaging member 77 is a portion engaging with the rib 68 of the inputted member 67.

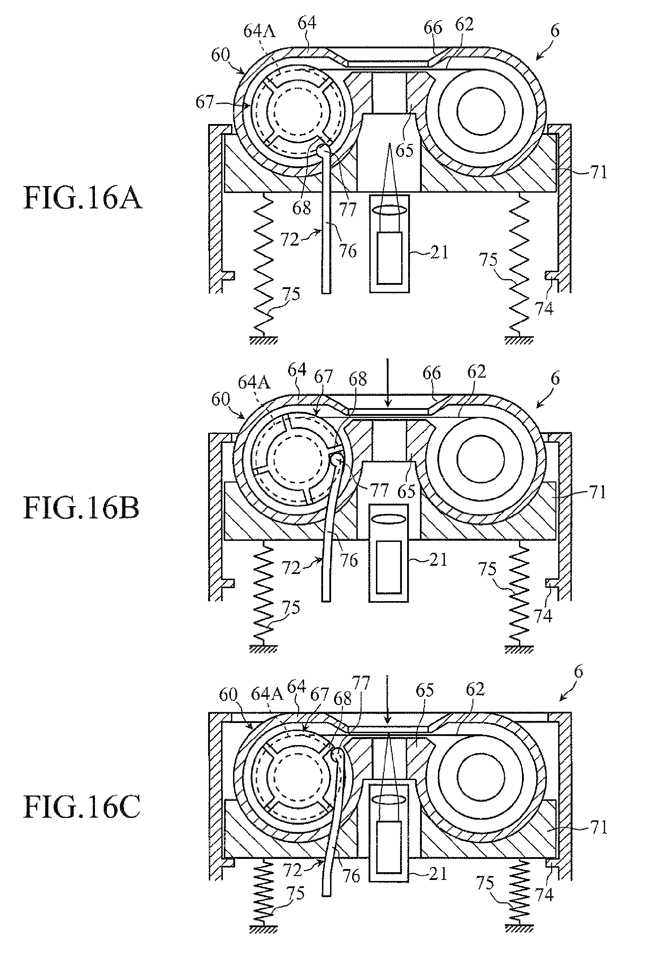

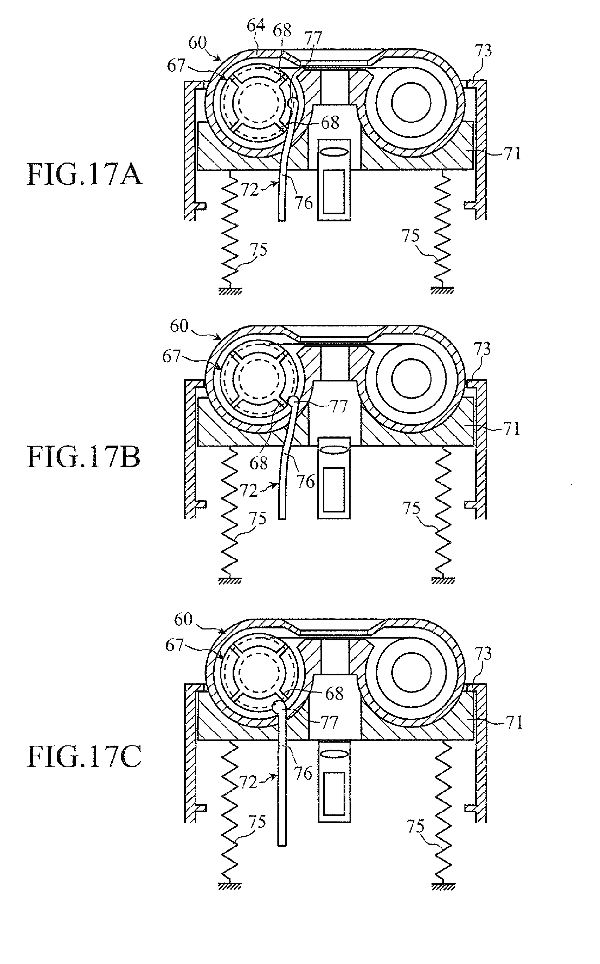

Next, operations of the perforator apparatus 6 will be described referring to FIGS. 16 and 17.

As shown in FIG. 16A through FIG. 16C, when a perforation is formed on the skin of a fingertip or the like, the cartridge 60 is moved downwardly until the supporting block 71 interferes with the stopper 74 in a state where skin is placed on the aperture 66 of the cartridge 60. On this occasion, since the inputted member 67 also moves downwardly, the rib 68 is pushed up by the inputting member 72. In this process, since the position of the engaging member 77 is displaced appropriately by elastic deformation of the elastic member 76 of the inputting member 72, the inputted member 67 is pushed up (turned) appropriately by the inputting member 72 without being hindered by other elements.

When the inputted member 67 is turned, the roll 64A is turned simultaneously and therefore, the film 62 is wound by the roll 64A. As a result, a portion positioned in the joining portion 65 in the film 72 is contained by the roll containing portion 66. The rotational angle of the inputted member 67 corresponding to the maximum movement distance of the supporting block 71 is set to 90 degrees in the example illustrated.

In the meantime, when the supporting block 71 is moved downwardly to the interference position with the stopper 74, laser light is output from the laser light oscillator 21. With this operation, laser light is applied to the skin, and the skin is then incised and bleeding of body fluid such as blood from the skin starts. Outputting of laser light from the laser light oscillator 21 may be performed automatically in the laser perforator apparatus 6 when the supporting block 71 is moved to the interference position with the stopper 74 or performed by the user who manipulates a button.

Meanwhile, as shown in FIG. 17A through FIG. 17C, when load input to the supporting block 71 (cartridge 60) is released, the coil spring 75 returns by elasticity and the supporting block 71 returns to a position that interferes with the stopper 73. On this occasion, although the inputting member 72 is displaced downwardly relative to the inputted member 67, since the inputted member 67 is rotatable in only one direction, the inputted member 67 does not turn. Further, since the inputting member 72 can cause appropriate positional displacement of the engaging member 77 by elastic deformation of the elastic member 76, after the supporting block 71 returns, engagement with the rib 68 is possible at a position where the engaging member 77 can push up the rib 68.

With the perforator apparatus 6 as mentioned above, a portion positioned at the joining portion 65 in the film 62 corresponds to the laser light transmission portion covering the laser light oscillator 21. Therefore, it is possible to protect the light outputting portion in the laser light oscillator 21 from smoke or the like by the film 62.

Further, by moving the film 62, in the perforator apparatus 6, a portion positioned at the joining portion 65 in the film 62 may be left as an unused part, which is hygienic.

Further, replacement of the cartridge 60 may be performed after the film 62 is used thoroughly and the film 62 need not be replaced every one-time perforation forming operation, thereby reducing burdens of the user.

For movement of the film 62 from the roll 63A to the roll 64A is not limited necessarily to one that utilizes the inputting member 72 and the inputted member 67. For example, the rolls 63A, 64A may be turned utilizing a known actuator. In this case, the supporting block 71 need not be configured to allow movement in up and down directions.

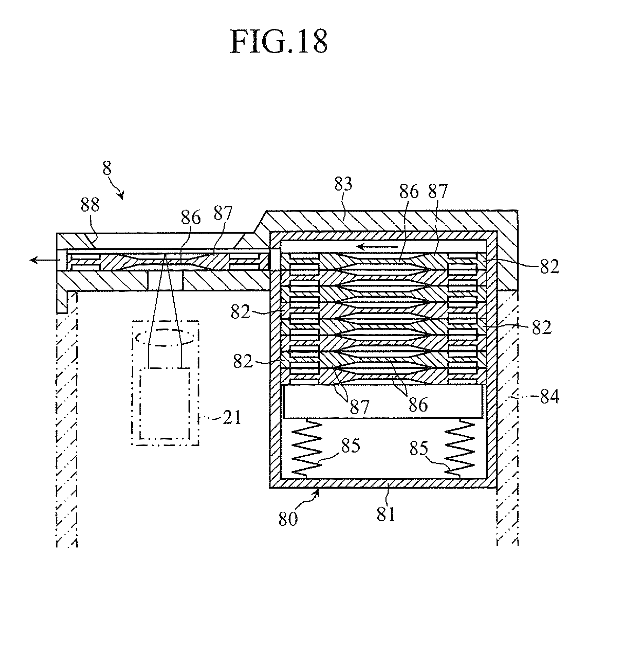

Next, a fourth embodiment of the present invention will be described referring to FIG. 18.

A perforator apparatus 8 shown in FIG. 18 protects the laser light oscillator 21 from smoke or the like using a cartridge 80.

The cartridge 80 contains a plurality of protection members 82 inside a case 81 and is contained in a housing 84 being retained by a cap 83.

The plurality of protection members 82 are biased upwardly by a coil spring 85 in a state of being laminated in a thickness direction inside the case 81 and can be moved to immediately above the laser light oscillator 21 (position of perforation forming) by moving the protection member 82 located at the uppermost. When the protection member 82 is taken out from the cartridge 80, remaining protection members 82 are moved upwardly by elastic force of the coil spring 85 and the protection member 82 located at the uppermost can now be taken out.

Movement of the protection member 82 from the case 81 to the position of perforation forming may be performed by an actuator utilizing a known mechanism, for example, a link mechanism, or utilizing magnetic force or electromagnetic force.

Each of the protection members 82 has a laser light transmission portion 86 and a ring pattern wall 87.

The laser light transmission portion 86 permits transmission of the laser light oscillated by the laser light oscillator 21 and is defined in a circular shape. The thickness of this laser light transmission portion 86 is set to, for example, from 0.1 to 0.5 mm. Meanwhile, the diameter of the laser light transmission portion 86 is set to, for example, from 2 to 5 mm. As a matter of course, the shape of the laser light transmission portion 86 is not limited to the circular shape and may take other shapes such as a rectangle.

The ring pattern wall 87 is a portion exposed by an aperture 88 of the cap 83 and is formed to enclose the laser light transmission portion 86. Since the ring pattern wall 87 is exposed from the aperture 88, when a fingertip or the like is placed on the aperture 88, the skin makes contact with the ring pattern wall 87.

With the perforator apparatus 8, the light outputting portion of the laser light oscillator 21 can be put into a state of being covered by the protection member 82 at perforation forming. Therefore, smoke generated when laser light is applied to the skin hardly reaches the light outputting portion of the laser light oscillator 21.

With the perforator apparatus 8, the laser light transmission portion 86 of the protection member 82 and the ring pattern wall 87 are exposed by the aperture 88 of the cap 83. Therefore, at laser light application, the skin such as a fingertip can be held in a state touched with the ring pattern wall 87 surrounding the laser light transmission portion 86. Meanwhile, the laser light is applied to the skin after passing through the laser light transmission portion 86 enclosed by the ring pattern wall 87. In other words, a perforation forming site (fluid bleeding portion) on the skin is a region enclosed by the ring pattern wall 87. For this reason, when bleeding of body fluid from the skin occurs due to laser light application, possibility of adhesion of the body fluid to the interior of the aperture 88 in the cap 83 is reduced.

With the perforator apparatus 8, when all the protection members 82 of the cartridge are used, the cartridge 80 may be replaced with a new one. Therefore, the user need not replace the protection members 82 every one-time perforation forming manipulation, thereby reducing burdens of the user.

The plurality of protection members 82 need not necessarily be retained by the cap 83 in the form of the cartridge 80 and such a constitution may be used that a space for containing a plurality of protection members 82 is provided to the cap 83 or the housing 84 and the plurality of protection members 82 are replenished all together.

## Claims

1. A laser perforator apparatus comprising:
a laser light oscillator for outputting laser light to be applied to skin; and
a protection member disposed on an optical path before laser light output from the laser light oscillator reaches the skin, wherein
the protection member covers a laser light outputting portion in the laser light oscillator and includes a plurality of laser light transmission portions permitting transmission of the laser light.

2. The laser perforator apparatus according to claim 1, wherein the protection member is configured to be movable in a direction intersecting a transmission direction of the laser light.

3. The laser perforator apparatus according to claim 2, wherein
the protection member is movable in a linear fashion, and
the plurality of laser light transmission portions are arranged in a row shape in a movement direction of the protection member.

4. The laser perforator apparatus according to claim 3, wherein
the protection members are arranged in the movement direction and have a plurality of individual regions having the laser light transmission portions, and
each of the individual regions is made separable at a fragile part provided between adjacent individual regions.

5. The laser perforator apparatus according to claim 4 further comprising a carrier device for movement of the protection member, wherein
the carrier device includes a rotating body having an engaging member for engaging with an engaged member formed to the protection member, and the individual region is separated at the fragile part by turning the rotating body with the engaging member being engaged with the engaged member.

6. The laser perforator apparatus according to claim 2, wherein the plurality of laser light transmission portions are disposed side by side on the same circumference.

7. The laser perforator apparatus according to claim 6, wherein
the protection members are formed in a rotatable disk shape, and
the plurality of laser light transmission portions are disposed side by side in a circumference direction of the protection member.

8. The laser perforator apparatus according to claim 2 further comprising a cap that movably retains the protection member, wherein the cap has an aperture for exposing the laser light transmission portion.

9. The laser perforator apparatus according to claim 8, wherein the protection member is provided on the periphery of the laser light transmission portion and further comprises a convex portion exposed via the aperture.

10. The laser perforator apparatus according to claim 9, wherein
the convex portion is formed in a ring pattern surrounding the laser light transmission portion, and
the aperture exposes the convex portion wholly.

11. The laser perforator apparatus according to claim 1, wherein the protection member is formed in a film shape that permits transmission of laser light.

12. The laser perforator apparatus according to claim 11, wherein the protection member is contained in a state of being wound in a case.

13. The laser perforator apparatus according to claim 12 further comprising a supporting member for supporting the case, wherein
the protection member is configured such that at least a part thereof is slid in a direction intersecting a movement direction of the supporting member by moving the supporting member.

14. A laser perforator apparatus comprising:
a laser light oscillator for outputting laser light to be applied to skin; and
a plurality of protection members for protecting the laser light oscillator, wherein
the protection member is configured to be movable from a position that does not cover a laser light outputting region in the laser light oscillator to a position that covers the outputting region.

15. The laser perforator apparatus according to claim 14, wherein the plurality of protection members are contained in a state of being formed in a plate-like shape and laminated in a thickness direction.

16. The laser perforator apparatus according to claim 15 further comprising a case containing the plurality of protection members, wherein the case is configured to be detachable.

17. The perforator apparatus according to claim 16 further comprising:
a housing containing the laser light oscillator; and
a cap covering the housing, wherein
the case is configured to be detachable with respect to the cap.

18. A protection member for a laser perforator apparatus including a laser light oscillator for outputting laser light to be applied to skin, the protection member being disposed on an optical path before laser light output from the laser light oscillator reaches the skin,
the protection member covering a laser light outputting portion in the laser light oscillator and comprising a plurality of laser light transmission portions permitting transmission of the laser light.

19. The protection member for a laser perforator apparatus according to claim 18, wherein the plurality of laser light transmission portions are disposed side by side on the same straight line.

20. The protection member for a laser perforator apparatus according to claim 19, comprising a plurality of individual regions having the laser light transmission portions, wherein
each of the individual regions is made separable at a fragile part provided between adjacent individual regions.

21. The protection member for a laser perforator apparatus according to claim 18, wherein the plurality of laser light transmission portions are disposed on the same circumference.

22. A cartridge used in a laser perforator apparatus including a laser light oscillator for outputting laser light to be applied to skin, comprising:
a protection members disposed on an optical path before laser light output from the laser light oscillator reaches the skin, wherein
the protection members are formed in a film shape that permits transmission of laser light.

23. The cartridge for a laser perforator apparatus according to claim 22, wherein the protection members are contained in a state of being wound in a case.

24. A cartridge used in a laser perforator apparatus including a laser light oscillator for outputting laser light to be applied to skin,
the cartridge containing a plurality of protection members for protection of the laser light oscillator.

25. The cartridge for a laser perforator apparatus according to claim 24, wherein the plurality of protection members are contained in a case in a state of being formed in a plate-like shape and laminated in a thickness direction.
